# EUROPEAN PATENT APPLICATION

(11) **EP 4 012 722 A1**
(43) Date of publication of application: **15.06.2022**
(21) Application number: 20213651.1
(22) Date of filing: 14.12.2020
(51) Int. Cl.: G16H 50/70, G16H 50/30, G16H 50/20

(54) **SLEEP QUALITY ANALYSIS**

(71) Applicant: Koninklijke Philips N.V., 5656 AG Eindhoven (NL)
(72) Inventor: MARGARITO, Jenny, 5656 AE Eindhoven (NL)
(74) Representative: Philips Intellectual Property & Standards

(57) **Abstract**

According to an aspect, there is provided a computer-implemented method for analyzing sleep quality. The method comprise receiving (S11) sleep data, relating to a user of a sleep monitoring device and receiving (S12) a user-specific sleep score from the user. The method further comprises extracting (S13) sleep metrics from the received sleep data. The method further comprises training (S14) a machine learning model, based on the sleep metrics and the user-specific sleep score, to calculate a sleep quality score. There is further provided a device (20) and a computer-readable medium (30) for analyzing sleep quality. In accordance with the present disclosure, sleep quality analysis may be improved.

## Description

### FIELD

The present invention relates to determining a sleep quality score. The sleep quality score may be determined based on analysis of sleep data and a user-specific sleep score.

### DESCRIPTION OF THE RELATED ART

Several types of consumer products to monitor sleep exist, such as smart watches, mobile apps and mattresses with embedded sensors. A reason for this large offering of sleep trackers is due to the increasing interest to know more about sleep. People look for factors that disrupt their sleep as well as recommendations for improving sleep quality, in order to feel more rested the following day and be able to perform at their peak. Systems for monitoring sleep may include electroencephalogram (EEG) sensor-based sleep monitoring devices, movement sensors, mattresses with embedded sensors, and many more.

Sleep may be categorized into either REM (rapid eye movement) sleep or NREM (non-rapid eye movement) sleep. NREM sleep may be split into different "stages", such as stage 1, stage 2 and stage 3, relating to progressively more "deep" sleep. Stage 3 NREM sleep is a deep form of sleep. NREM sleep is known to have beneficial effects on brain function, such as improved memory function. In order to determine whether a person is in stage 3 NREM sleep, sensors may be arranged so as to monitor a user and measurements may be taken and monitored for characteristics indicative of the different stages or categories of sleep. Sometimes however there may be discrepancies between the measurements and identified characteristics of a user's sleep, and the user's own perception of the quality of their sleep. A user's own perception may for example take into account tiredness, alertness and energy levels during the following day. Therefore, there is a need for a method to improve the assessment of sleep quality, based on the measurements taken.

### SUMMARY

According to an embodiment of an aspect, there is provided a computer-implemented method for analyzing sleep quality. The method comprises receiving sleep data. The sleep data relates to a user of a sleep monitoring device. The method further comprises receiving a user-specific sleep score from the user. The method further comprises extracting sleep metrics from the received sleep data. The method further comprises training a machine learning model, based on the sleep metrics and the user-specific sleep score, to calculate a sleep quality score.

According to an embodiment of a further aspect, there is provided a device for analyzing sleep quality. The device comprises a receiver to receive sleep data, relating to a user of a sleep monitoring device, and to receive a user-specific sleep score from the user. The device further comprises a processor to extract sleep metrics from the received sleep data; and train a machine learning model, based on the sleep metrics and the user-specific sleep score, to calculate a sleep quality score.

According to an embodiment of a further aspect, there is provided a non-transitory computer-readable medium storing a program which, when executed on a computer, is configured to cause the computer to perform a process. The process comprises receiving sleep data, relating to a user of a sleep monitoring device. The process further comprises receiving a user-specific sleep score from the user. The process further comprises extracting sleep metrics from the received sleep data. The process further comprises training a machine learning model, based on the sleep metrics and the user-specific sleep score, to calculate a sleep quality score.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a flowchart of an example of a method in accordance with an embodiment;
Fig. 2 is another flowchart of an example of a method in accordance with an embodiment;

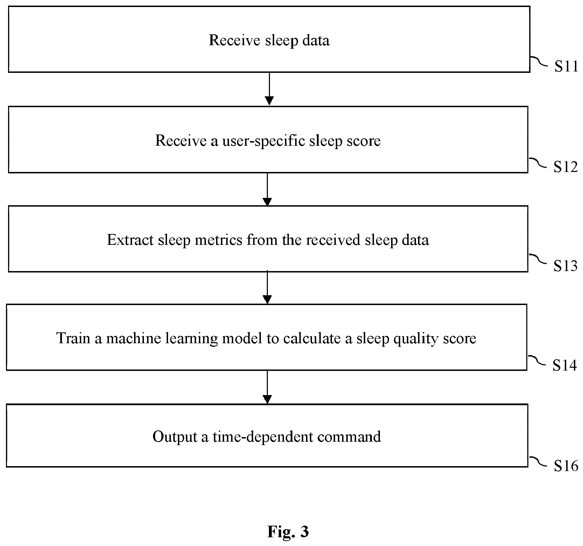

Fig. 3 is another flowchart of an example of a method in accordance with an embodiment;
Fig. 4 is a simplified schematic of an example of a device in accordance with an embodiment;
Fig. 5 is another simplified schematic of an example of a device in accordance with an embodiment;
Fig. 6 is another simplified schematic of an example of a device in accordance with an embodiment; and
Fig. 7 is an example of a medium in accordance with an embodiment.

### DETAILED DESCRIPTION

Several types of consumer products to monitor sleep exist: smart watches, smartphone apps, headbands, and sleep mattresses equipped with sensors, for example. Each of these monitoring devices provides objective data relating to the characteristics of the user's sleep. Further, different technologies can be used to detect a person's sleep characteristics (for example sleep duration, sleep stage durations, sleep efficiency), such as actigraphy, ballistocardiography, photoplethysmography and electroencephalography. Products or technologies may have their data compared with data taken via polysomnography (PSG), which is considered to be the gold standard for sleep monitoring. The accuracy of each of the different technologies and consumer products may vary and there may be differences between the readings taken for the same sleeping activity. For example, wrist worn devices may overestimate sleep duration compare to headbands.

Several standard measures may be used to objectively quantify a person's sleep quality, such as sleep efficiency or duration of deep sleep. However, objective sleep quality metrics may not reflect the person's perception (e.g., sleep efficiency might be very high but the user does not feel rested). In fact, for some users the duration of deep sleep (known as N3 stage or stage 3 NREM sleep) might have higher impact on their perception and physiological feeling than the sleep efficiency. In such scenario, any insight provided to the user about behavioral changes to improve their sleep quality may not be trusted and may be considered inaccurate. As mentioned above, a further problem is that different types of sensors/technologies used to monitor a person's sleep architecture can provide a different number for the same metric. For example, a technology that tends to overestimate sleep time will provide higher sleep efficiency than a technology that tends to underestimate it.

Therefore, there is provided a method to train a machine learning model, such as an artificial neural network, to find the sleep architecture metrics that better describe the subjective sleep quality and provide a sleep quality score that more closely matches the user's perception and physical condition. In this way, based on the sleep quality score, it may be possible to provide more useful recommendations for improving the sleep quality.

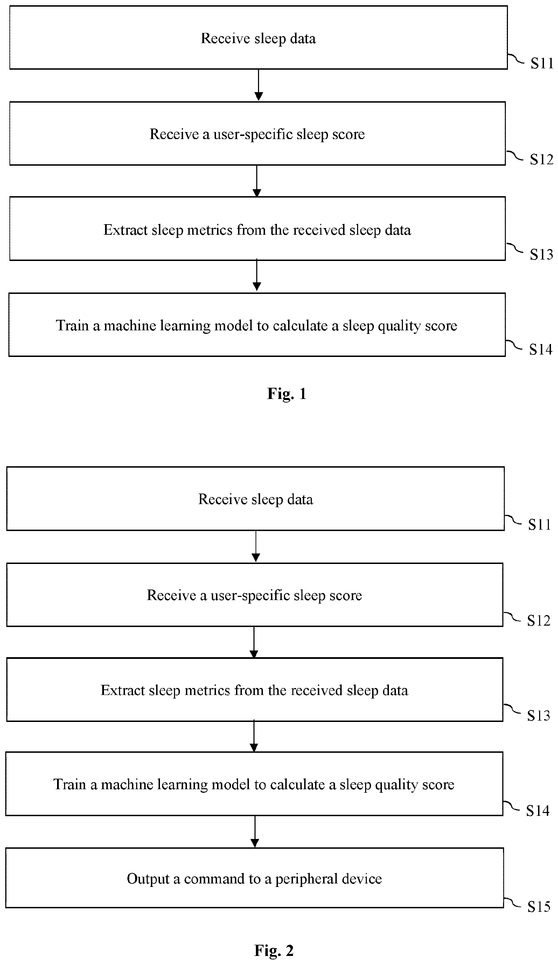

In an example, as shown in Fig. 1, there is provided an embodiment of a computer-implemented method for analyzing sleep quality. The method comprise receiving S11 sleep data, relating to a user of a sleep monitoring device and receiving S12 a user-specific sleep score from the user. The method further comprises extracting S13 sleep metrics from the received sleep data. The method further comprises training S14 a machine learning model, based on the sleep metrics and the user-specific sleep score, to calculate a sleep quality score.

The method may be used to train a machine learning model, such as a neural network, to more accurately estimate the quality of a user's sleep. A sleep quality score may then be presented to the user for comparison with their subjective sleep score. A user-specific sleep score may be an estimation, by the user, of sleep quality based on factors such as energy levels on the day following the sleep activity, alertness, tiredness, etc. Such factors may be subjective or may be measured using established tests, such as reaction timing and physical exercise evaluation. A user-specific sleep score may be produced every day or after every sleep activity. A sleep questionnaire may be used for subjective sleep quality assessment. Sleep metrics may include categories which are related to sleep quality; total sleep time, sleep efficiency and so forth. More detail on the sleep metrics is given below.

The sleep data may be received from the sleep monitoring device directly or via another device. The sleep data may include one or more categories of data, allowing for extraction of one or more sleep metrics. One or more sleep metrics may be extracted from a single type of sleep data. Before being compared, the sleep metrics and the user-specific sleep score may be scaled or discretized, to allow for more useful comparison.

In some examples, training the machine learning model may comprise comparing the sleep metrics and the user-specific sleep score. Such a comparison may comprise determining ranges for each sleep metric which correlate to a particular user-specific sleep score. Based on the comparison and a type of each sleep metric, the method may further comprise determining a weight to apply to each sleep metric and calculating the sleep quality score based on the sleep metrics and applied weights. A weight may represent a significance of the sleep metric to which it is applied. That is to say, some sleep metrics may be more relevant than others, and therefore the applied weight may be used to reflect this. In some examples, sleep metrics having a weight lower than a predetermined threshold value are excluded from consideration and the sleep quality score is calculated based on remaining sleep metrics.

A machine learning model may be any suitable model for the processing of the relevant sleep metrics and user-specific sleep score. Moreover, a specific model may be selected based on the sleep metrics. In more detail, the sleep metrics deemed to be the most relevant in assessing the sleep quality of the sleep activity, for example having the highest weighting, may be used to select an appropriate model to be trained for the purpose of calculating a sleep quality score. The sleep quality score may indicate sleep quality of the sleep activity to which the sleep data, sleep metrics and user-specific sleep score relates. The model may process sleep metrics and user-specific sleep scores relating to many sleep activities over time in order to provide an ever-more accurate sleep quality score. With enough training, the model may be able to accurately estimate or match the user-specific sleep score. The model may be trained in a training phase. Following the training phase, the same model may then be used in an implementation phase. In the implementation phase, similar sleep data or sleep metrics may be input into the model, but without the user-specific sleep score, in order to output a sleep quality score.

In an example, the machine learning model may be selected from a plurality of machine learning models, based on the sleep metrics and applied weights. A database of models may be provided storing a plurality of models, each structured to process different types of sleep metrics. Each model may be a specific type and may have specific characteristics suited to the analysis of one or more of the sleep metrics. In an example, the sleep data may be in the form of a hypnogram, and the model may be programmed to correlate hypnogram data with the user specific sleep score by identifying the characteristics of the user's sleep that most match the user's subjective impression of the quality of their sleep. Sleep metrics that are extracted from the received sleep data may include (with the units given in parentheses): sleep efficiency (%), percentage of sleep in REM stage (%), number of minutes in REM stage (min), percentage of sleep in N3 stage (%), number of minutes in N3 stage (min), sleep onset latency (min), wake after sleep onset (min), total sleep time (min) and number of sleep stage transitions (#). Other sleep metrics are also envisaged. Sleep metrics deemed not to correlate with the user-specific sleep score may be excluded from the sleep quality score calculation.

In a further example, each weight may be applied to the associated sleep metric, based on detected sleep periods. Sleep periods may be identified from the received sleep data. Each sleep period may relate to a different sleep stage, as set out above. Each sleep period may have a different reliability associated with the sleep data. Therefore, sleep data from one type of sensor may be more reliable for a particular sleep stage than another. For example, electroencephalogram (EEG) readings taken during a sleeping activity may give a reliable indication of the sleep stage of the user. If however the user moves and disrupts the electrodes used to detect the EEG signal, from that point on the EEG readings may be considered unreliable and may correspondingly be given a low weighting.

In some examples, the sleep monitoring device is associated with one or more types of sleep data. A sleep monitoring device may include for example a wearable device including sensors to detect any one or more of movement, blood pressure, pulse rate and skin temperature. In another example, the sleep monitoring device may be a headband including an EEG sensor, a skin perspiration sensor and a skin temperature sensor. Each type of sleep data may be associated with a different sleep metric and therefore may be given a different weight.

In an example, the method may further comprise outputting S15 a command to a peripheral device based on the sleep metrics and applied weights. A peripheral device may include a wearable device, such as an activity monitor, or a virtual assistant or any other suitable device.

The sleep metrics and the user-specific sleep score may be discretized prior to the comparing. Further, the sleep metrics and the user-specific sleep score may be scaled prior to the comparing. In order to aid the comparison, the sleep metrics and the user-specific sleep score may be converted into more useful values for the purpose of a comparison. In other words, a sleep metric, such as sleep efficiency for example may be presented as a percentage, and the user-specific sleep score may be converted into a percentage from whatever value or scale the user inputs their sleep score as.

Further, data or metrics may be discretized to obtain lower resolution scores, which may simplify, and aid, the processing carried out by the machine learning model. For example a user-specific sleep score within a range of 1 to 10 may be discretized into three classes covering 1) 1 to 3 2) 4 to 7 3) 8 to 10. The thresholds defining the classes may be heuristically defined or may be learnt based on the collected data, metrics and scores. For instance, thresholds for the score discretization may be derived by the distribution of collected scores. To discretize the sleep score in two classes, 0 and 1, referring to bad and good quality sleep, respectively, the percentiles of the subjective sleep scores collected during the monitoring time may be used to define the threshold between 0 and 1 as follows: If the user-specific sleep score > 80^{th} percentile of subjective sleep score distribution, then assign subjective score to class 1; otherwise assign subjective score to class 0. By discretizing the sleep score, the training of the model may be addressed either as a regression (because classes are ordinal) or a classification task. Different types of learning algorithms may be used by the machine learning model. In case of a regression task, linear/non-linear regression, Decision Tree Regressors and Random Forest Regressors may all be considered as options. For classification tasks, Logistic Regression, Support Vectors Machines and Neural Networks may be used. Feature selection techniques may also be applied to select the most relevant sleep metrics for a specific user. Such selection could be applied before the training of the model or embedded in the model training.

In an example, the method may further comprise outputting S16 a time-dependent command, based on the sleep metrics and applied weights. A time-dependent command may be output to assist the user in improving their sleep quality for the following night. A time-dependent command may take the form of a signal to a wearable activity monitor to remind the user to exercise earlier in the day, to wake up at a specific time or to go to sleep at a specific time. Such a command could further control other devices such as home lighting to turn on or off, based on the command.

Through correlation analysis, sleep metrics that most closely correlated with a person's subjective sleep score are different for each subject. Isolating metrics that correlate to the user-specific sleep score improves the quality of the analysis by eliminating metrics which do not reflect the real perception of the user, and then providing outputs, such as advice on improving sleep quality, based on the isolated metrics.

In some examples, the method detailed above may be carried out by a program, which may be stored on any suitable storage medium, such that when the program is executed on a suitable computer processor, a process is carried out according to the method. Further, the method may be performed on any suitable device, an example of which is detailed below.

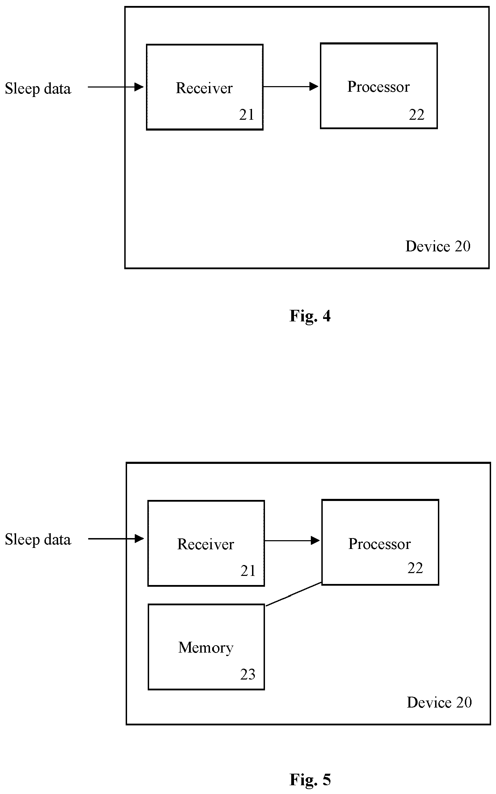

In an example, as shown in Fig. 4, there is provided an embodiment of a device 20 for analyzing sleep quality. The device 20 comprises a receiver 21 to receive sleep data, relating to a user of a sleep monitoring device, and to receive a user-specific sleep score from the user. The device 20 further comprises a processor 22 to extract sleep metrics from the received sleep data; compare the sleep metrics and the user-specific sleep score; and train a machine learning model, based on the sleep metrics and the user-specific sleep score, to calculate a sleep quality score.

The sleep monitoring device may for example be separate from the device 20, or included in the device 20. The sleep monitoring device be a wearable device or any other device for detecting physiological readings relating to the sleep characteristics of the user. The sleep monitoring device may for example be an electroencephalogram detector including electrodes to be positioned appropriately on the user. The processor 22 may be any suitable computing device and may comprise a neural network on which the machine learning model may be executed. The receiver 21 may be any device suitable for receiving sleep data. The weight may for example give the associated sleep metric a relative significance. Weights may be used to eliminate some sleep metrics from use in later analysis or for machine learning model training.

In some examples, the processor may be configured to train the machine learning model by comparing the sleep metrics and the user-specific sleep score and, based on the comparison and a type of each sleep metric, determining a weight to apply to each sleep metric and calculating the sleep quality score based on the sleep metrics and applied weights.

In an example, as shown in Fig. 5, the device 20 may further comprise a memory 23 to store the sleep data, sleep metrics and the applied weights. The memory 23 may be any suitable storage device for storing the sleep data, sleep metrics, the applied weights and any other data or metadata useful for analyzing sleep quality and training a machine learning model.

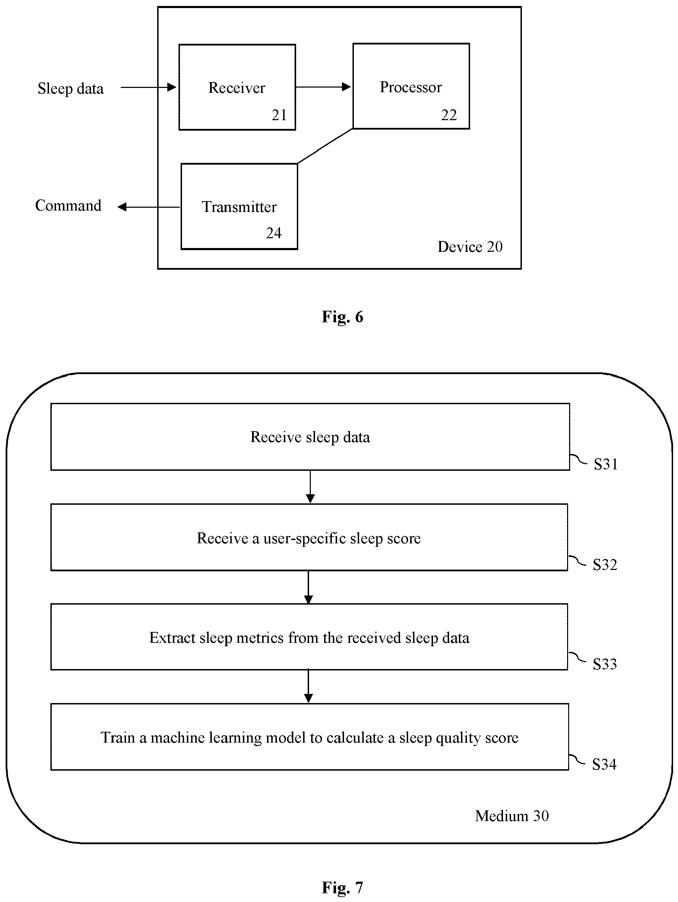

In an example, as shown in Fig. 6, the device 20 may further comprise a transmitter 24 to, based on the sleep metrics and user-specific sleep score, transmit a command to a peripheral device. The transmitter 24 may be any suitable communication device to transmit commands. In some examples, the command may be a time-dependent command, such that it is transmitted in at a specific time or so that the instructions in the command are to be carried out at a specific time. In some examples, the device 20 may be a wearable device.

In an example, as shown in Fig. 7, there is provided an embodiment of a non-transitory computer-readable medium 30 storing a program which, when executed on a computer, is configured to cause the computer to perform a process. The process comprises receiving S31 sleep data, relating to a user of a sleep monitoring device. The process further comprises receiving S32 a user-specific sleep score from the user. The process further comprises extracting S33 sleep metrics from the received sleep data. The process further comprises training S34 a machine learning model, based on the sleep metrics and the user-specific sleep score, to calculate a sleep quality score.

In some examples, the machine learning model is selected from a plurality of machine learning models, based on the sleep metrics and applied weights. The process may further comprise outputting, based on the sleep metrics and associated weight, a command to a peripheral device.

Examples in the present disclosure can be provided as methods, systems or machine readable instructions, such as any combination of software, hardware, firmware or the like. Such machine readable instructions may be included on a computer readable storage medium (including but is not limited to disc storage, CD-ROM, optical storage, etc.) having computer readable program codes therein or thereon.

The present disclosure is described with reference to flow charts and block diagrams of the method, devices and media according to examples of the present disclosure. Although the flow diagrams described above show a specific order of execution, the order of execution may differ from that which is depicted. Blocks described in relation to one flow chart may be combined with those of another flow chart. It shall be understood that each flow and block in the flow charts and block diagrams, as well as combinations of the flows and diagrams in the flow charts and block diagrams can be realized by machine readable instructions.

The machine readable instructions may, for example, be executed by a general purpose computer, a special purpose computer, an embedded processor or processors of other programmable data processing devices to realize the functions described in the description and diagrams. In particular, a processor or processing apparatus may execute the machine readable instructions. Thus functional modules of the apparatus and devices may be implemented by a processor executing machine readable instructions stored in a memory, or a processor operating in accordance with instructions embedded in logic circuitry. The term 'processor' is to be interpreted broadly to include a CPU, processing unit, ASIC, logic unit, or programmable gate array etc. The methods and functional modules may all be performed by a single processor or divided amongst several processors.

Such machine readable instructions may also be stored in a computer readable storage that can guide the computer or other programmable data processing devices to operate in a specific mode.

Such machine readable instructions may also be loaded onto a computer or other programmable data processing devices, so that the computer or other programmable data processing devices perform a series of operations to produce computer-implemented processing, thus the instructions executed on the computer or other programmable devices realize functions specified by flow(s) in the flow charts or block(s) in the block diagrams.

Further, the teachings herein may be implemented in the form of a computer software product, the computer software product being stored in a storage medium and comprising a plurality of instructions for making a computer device implement the methods recited in the examples of the present disclosure.

The features of any dependent claim may be combined with the features of any of the independent claims or other dependent claims.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer-implemented method for analyzing sleep quality, the method comprising:
receiving (S11) sleep data, relating to a user of a sleep monitoring device;
receiving (S12) a user-specific sleep score from the user; extracting (S13) sleep metrics from the received sleep data; and
training (S14) a machine learning model, based on the sleep metrics and the user-specific sleep score, to calculate a sleep quality score.

2. The method of claim 1, wherein
training the machine learning model comprises comparing the sleep metrics and the user-specific sleep score and, based on the comparison and a type of each sleep metric, determining a weight to apply to each sleep metric and calculating the sleep quality score based on the sleep metrics and applied weights.

3. The method of claim 2, wherein
sleep metrics, having a weight lower than a predetermined threshold value, are excluded and the sleep quality score is calculated based on remaining sleep metrics.

4. The method of any preceding claim, wherein
the machine learning model is selected from a plurality of machine learning models, based on the sleep metrics and applied weights.

5. The method of any preceding claim, wherein
each weight is applied to the associated sleep metric, based on detected sleep periods.

6. The method of any preceding claim, wherein
the sleep monitoring device is associated with one or more types of sleep data.

7. The method of any preceding claim, further comprising:
outputting (S15), based on the sleep metrics and applied weights, a command to a peripheral device.

8. The method of any preceding claim, wherein
the sleep metrics and the user-specific sleep score are discretized prior to the comparing.

9. The method of any preceding claim, further comprising:
outputting (S16) a time-dependent command, based on the sleep metrics and applied weights.

10. A device (20) for analyzing sleep quality, the device (20) comprising:
a receiver (21) configured to receive sleep data, relating to a user of a sleep monitoring device, and to receive a user-specific sleep score from the user;
a processor (22) configured to:
extract sleep metrics from the received sleep data;
compare the sleep metrics and the user-specific sleep score; and
train a machine learning model, based on the sleep metrics and the user-specific sleep score, to calculate a sleep quality score.

11. The device (20) of claim 10, wherein
the processor is configured to train the machine learning model by comparing the sleep metrics and the user-specific sleep score and, based on the comparison and a type of each sleep metric, determining a weight to apply to each sleep metric and calculating the sleep quality score based on the sleep metrics and applied weights.

12. The device (20) of claim 11, further comprising:
a memory (23) configured to store the sleep data, sleep metrics and the applied weights.

13. The device (20) any of claims 10 to 12, further comprising:
a transmitter (24) configured to, based on the sleep metrics and user-specific sleep score, transmit a command to a peripheral device.

14. The device (20) of any of claims 10 to 13, wherein
the device (20) is a wearable device.

15. A non-transitory computer-readable medium (30) storing a program which, when executed on a computer, is configured to cause the computer to perform a process comprising:
receiving (S31) sleep data, relating to a user of a sleep monitoring device;
receiving (S32) a user-specific sleep score from the user;
extracting (S33) sleep metrics from the received sleep data; and
training (S34) a machine learning model, based on the sleep metrics and the user-specific sleep score, to calculate a sleep quality score.
